**Europäisches Patentamt**

**European Patent Office**

**Office européen des brevets**

(19)

(11) Veröffentlichungsnummer : **0 324 419 B1**

(12) **EUROPÄISCHE PATENTSCHRIFT**

(45) Veröffentlichungstag der Patentschrift :
**08.04.92 Patentblatt 92/15**

(21) Anmeldenummer : **89100304.8**

(22) Anmeldetag : **10.01.89**

(51) Int. Cl.⁵ : **C07C 53/124, C07C 51/44**

(54) **Verfahren zur kontinuierlichen Herstellung von Isobuttersäure.**

(30) Priorität : **11.01.88 DE 3800473**

(43) Veröffentlichungstag der Anmeldung :
**19.07.89 Patentblatt 89/29**

(45) Bekanntmachung des Hinweises auf die
Patenterteilung :
**08.04.92 Patentblatt 92/15**

(84) Benannte Vertragsstaaten :
**AT BE CH DE ES FR GB IT LI NL SE**

(56) Entgegenhaltungen :
**EP-A- 0 031 886**

(56) Entgegenhaltungen :
**WO-A-83/02940
KIRK-OTHMER, ENCYCLOPEDIA OF CHEMI-
CAL TECHNOLOGY; 3rd edition, vol. 7, page
870**

(73) Patentinhaber : **RÖHM GMBH
Kirschenallee
W-6100 Darmstadt (DE)**

(72) Erfinder : **Ruppert, Wolfgang, Dr. Ing.
Am Mühlgraben 9
W-6101 Bickenbach (DE)**
Erfinder : **Siegert, Hermann-Josef, Dr.
Burkhardtstrasse 38
W-6104 Seeheim-Jugenheim (DE)**

## Beschreibung

Die Erfindung betrifft ein Verfahren zur kontinuierlichen Herstellung von Isobuttersäure durch Koch'sche Synthese aus etwa stöchiometrischen Mengen an Propylen, Kohlenmonoxid und Wasser oder einem Alkohol in flüssigem Fluorwasserstoff als Koch'schem Katalysator unter Druck.

Die Grundzüge eines solchen Verfahrens sind aus der EP-B 31 886 und WO 83/02940 bekannt. In EP-B 31 886 wird die Aufarbeitung des Reaktionsgemisches in zwei Destillationsstufen vorgeschlagen. In der ersten Stufe sollen Fluorwasserstoff, nicht umgesetztes Propylen und niedrigsiedende Nebenprodukte, wie Isopropylalkohol, Isopropylfluorid, Isobuttersäurefluorid, Isobuttersäure-isopropylester und Diisopropyläther, über Kopf abdestilliert und in den Reaktor zurückgeführt werden. Für die Auftrennung des zurückbleibenden Gemisches aus Isobuttersäure, Oligomeren des Propylens und anderen Hochsiedern ist eine zweite Destillationskolonne vorgesehen, aus der Isobuttersäure als Kopfprodukt abgenommen wird. Ebenso werden in WO 83/02940 zwei Destillationsstufen als unerläßlich angesehen.

Für die technische Durchführung eines solchen kontinuierlichen Verfahrens unter wirtschaftlichen Bedingungen stellen sich Probleme, die sich aus der Forderung nach hoher Reinheit der Verfahrensprodukte, also nach einer sehr weitgehenden Auftrennung von Reaktionsgemischen einerseits und aus der Forderung nach möglichst geringem Anlagen- und Energieeinsatz andererseits ergeben.

Grundsätzlich ist die Forderung nach hoher Reinheit der aus einem Reaktionsgemisch abzutrennenden Bestandteile leicht zu erfüllen, wenn man für jeden Bestandteil eine geeignete Abtrennungsstufe vorsieht. So lassen sich Gemische aus einer Vielzahl von flüchtigen Bestandteilen durch Destillation schrittweise in hoch- und niedrigsiedende Komponenten zerlegen, wobei man die geforderte Reinheit durch eine geeignete Bodenzahl der Auf- und Abtriebssäule und durch ein geeignetes Rücklaufverhältnis gewährleisten kann. Das führt aber bei Gemischen aus zahlreichen Bestandteilen und bei hohen Reinheitsanforderungen zu einem Aufwand an Apparaten und an Wärmeenergie, der wirtschaftlich nicht tragbar ist.

## Aufgabe und Lösung

Es bestand daher die Aufgabe, bei einem kontinuierlichen Verfahren der eingangs beschriebenen Art mit nur einer Destillationsstufe auszukommen und dennoch eine von Fluorwasserstoff freieund nur geringfügig mit Hochsiedern verunreinigte Isobuttersäure zu gewinnen.

Erfindungsgemäß wird die Aufgabe dadurch gelöst, daß das Reaktionsgemisch nach erfolgter Umsetzung entspannt, die Hauptmenge der sich gasförmig abtrennenden Bestandteile abgeschieden und die zurückbleibende flüssige Phase in den mittleren bis oberen Bereich einer Destillationskolonne eingeleitet wird und daß die Isobuttersäure von einem Boden entnommen wird, an dem die stationären Konzentrationen von Fluorwasserstoff unter 100 ppm und von Stoffen, die höher als Isobuttersäure sieden, unter 1 Gew.-% liegen, wobei die Sumpftemperatur oberhalb des Siedepunktes der Isobuttersäure und die Kopftemperatur nahe beim Siedepunkt des Fluorwasserstoffes, jeweils unter dem dort herrschenden Druck, gehalten werden.

## Ausführung der Erfindung

Die Koch'sche Synthese von Isobuttersäure in Fluorwasserstoff als Koch'schem Katalysotor verläuft in einem heterogenen Reaktionsgemisch unter Druck. Das Reaktionsgemisch besteht aus einer flüssigen Phase, die zu einem wesentlichen Teil aus Fluorwasserstoff besteht. Die Gasphase enthält vor allem Kohlenmonoxid und einen dem Dampfdruck bei der Reaktionstemperatur entsprechenden Anteil an Fluorwasserstoff. Der Gehalt an Propylen ist in der Regel in beiden Phasen äußerst gering. Der Druck kann z.B. 50 bis 400 bar betragen. Diese Zusammensetzung ist unabhängig davon, ob Propylen, Kohlenmonoxid, Wasser und Fluorwasserstoff kontinuierlich als solche oder in Form von binären oder ternären Additionsprodukten, wie Isopropanol oder Isopropylformiat, kontinuierlich zugeführt werden.

Im kontinuierlichen Verfahren wird ein Teil des Reaktionsgemisches zweckmäßig an einer Stelle im unteren Bereich des Reaktors abgenommen, wo der Anteil an dispergierter Gasphasegering ist, so daß es praktisch nur aus der Flüssigphase besteht. Beim Entspannen trennt sich das Gemisch spontan in eine Gasphase, die überwiegend aus Fluorwasserstoff, gelöstem Kohlenmonoxid und zu einem kleinen Anteil aus gasförmigen Verunreinigungen und Niedersiedern besteht, und eine Flüssigphase auf. Sie enthält neben Isobuttersäure und Fluorwasserstoff als Hauptkomponenten alle Hochsieder und den der Löslichkeit entspechenden Anteil der in der Gasphase vorkommenden Komponenten.

Die zurückbleibende Flüssigphase wird erfindungsgemäß in einer Destillationkolonne in mindestens drei Teilströme zerlegt, nämlich Fluorwasserstoff als Kopfprodukt, hochsiederhaltige Isobuttersäure als Sumpfprodukt und reine Isobuttersäure als Seitenabstrom.

Der Kolonnenkopf wird durch an sich bekannte Maßnahmen der kontinuierlichen Destillationsführung nahe beim Siedepunkt des Fluorwasserstoffes bei dem am Kolonnenkopf herrschenden Druck gehalten. Daher wird am Kolonnenkopf nahezu reiner Fluorwasserstoff, gegebenenfalls zusammen mit Spuren von gasförmigen Verunreinigungen und geringen Mengen Isopropylfluorid abgenommen. Die Abweichung vom Siedepunkt des reinen Fluorwasserstoffs hängt von dem Anteil dieser Nebenbestandteile ab und beträgt im allgemeinen höchstens 1 oder 2 Kelvingrade. Der Druck am Kolonnenkopf wird vorzugsweise auf 0,5 bis 10 bar eingestellt, so daß sich eine Kopftemperatur von 0 bis 90 Grad C ergibt.

Der Kolonnensumpf wird bei einer Temperatur oberhalb des Siedepunktes der Isobuttersäure bei dem dort herrschenden Druck gehalten. Der Siedepunkt der Isobuttersäure in dem zuvor genannten Druckbereich liegt zwischen 135 und 248 Grad C. Die Sumpftemperatur liegt vorzugsweise etwa 30 bis 90 K höher. Im Sumpf befindet sich ein Gemisch aus einem überwiegenden Anteil Isobuttersäure und einem kleineren Anteil, beispielsweise 0,01 bis 0,5 Gew.-%, an Hochsiedern. Das Sumpfprodukt wird in einer solchen Menge abgezogen, daß sein Niveau und sein Gehalt an Hochsiedern etwa konstant bleiben.

Der Zulauf in die Destillationskolonne erfolgt im mittleren bis oberen Bereich. Dabei stellt sich in einem Bereich zwischen dem Zulauf und dem Sumpf eine Zone ein, in der sich nahezu reine Isobuttersäure befindet. Sie wird von einem Boden in diesem Bereich flüssig abgezogen. Die Ausbildung dieser Zone ist darauf zurückzuführen, daß in dem Reaktionsgemisch nur wenig oder keine Bestandteile mit einem nahe bei Isobuttersäure liegendem Siedepunkt auftreten. Dadurch sind in der erwähnten Zone die Niedersieder praktisch vollkommen entfernt, und der Hochsieder entsprechend dem Verhältnis vom Rücklaufstrom zum Zulaufstrom herabgesetzt.

Wenn im Reaktor Reaktionsbedingungen nach der Lehre der EP-B 31 886 eingehalten werden, entstehen nur verschwindend geringe Mengen an Hochsiedern. Charakteristisch für dieses Verfahren ist eine Umsetzungstemperatur von 80 bis 160 Grad C, ein Propylengehalt unter 1 Gew.-%, ein Wassergehalt unter 5 Mol-% und eine Verweilzeit der flüssigen Phase unter 20 Minuten. Man erhält in diesem Falle mit dem erfindungsgemäßen Zerlegungsverfahren eine Isobuttersäure von entsprechend hoher Reinheit. Der Gehalt an Fluorwasserstoff liegt unter 100 ppm und der Gehalt an Hochsiedern unter 1 Gew.-%; erreichbar sind z.B. 100 bis 500 ppm.

## Patentansprüche

1. Verfahren zur kontinuierlichen Herstellung von Isobuttersäure durch koch'sche Synthese aus etwa stöchiometrischen Mengen an Propylen, kohlenmonoxid und Wasser oder einem Alkohol in flüssigem Fluorwasserstoff als koch'schem katalysator unter Druck,
dadurch gekennzeichnet,
daß das Reaktionsgemisch nach erfolgter Umsetzung entspannt, die Hauptmenge der sich gasförmig abtrennenden Bestandteile abgeschieden und die zurückbleibende flüssige Phase in den mittleren bis oberen Bereich einer Destillationskolonne eingeleitet wird und daß die Isobuttersäure von einem Boden entnommen wird, an dem die stationären konzentrationen von Fluorwasserstoff unter 100 ppm und von Stoffen, die höher als Isobuttersäure sieden, unter 1 Gew.-% liegen, wobei die Sumpftemperatur oberhalb des Siedepunktes der Isobuttersäure und die kopftemperatur nahe beim Siedepunkt des Fluorwasserstoffes, jeweils unter dem dort herrschenden Druck, gehalten werden.

## Revendications

1. Procédé de préparation continue d'acide isobutyrique par synthèse de Koch à partir de quantités à peu près stoechiométriques de propylène, de monoxyde de carbone et d'eau ou d'un alcool, dans de l'acide fluorhydrique liquide servant, de catalyseur de Koch et sous pression,
caractérisé en ce
qu'après l'achèvement de la réaction, le mélange réactionnel est détendu, la majeure partie des composants qui se dégagent à l'état gazeux est séparée et la phase liquide restante est introduite dans la région moyenne à supérieure d'une colonne de distillation, et en ce que l'acide isobutyrique est prélevé à partir d'un plateau au niveau duquel la concentration stationnaire d'acide fluorhydrique se situe au-dessous de 100 ppm et celle de substances dont le point d'ébullition est supérieur à celui de l'acide isobutyrique se situe au-dessous de 1% en poids, la température de bas de colonne étant maintenue au-dessus du point d'ébullition de l'acide isobutyrique et la température en tête étant maintenue au voisinage du point d'ébullition de l'acide fluorhydrique, chaque fois sous la pression qui règne à ce niveau.

**Claims**

1. Process for the continuous preparation of isobutyric acid by Koch's synthesis from approx. stoichiometric amounts of propylene, carbon monoxide and water or an alcohol in liquid hydrogen fluoride as Koch's catalyst under pressure,
characterised in that,
after the reaction has taken place, the reaction mixture is expanded, the majority of the matter separating in gaseous form is isolated and the remaining liquid phase is introduced into the middle to upper region of a distillation column, and that isobutyric acid is removed from a bottom region, at which the stationary concentrations of hydrogen fluoride are 100 ppm and those of substances boiling at a higher temperature than isobutyric acid are below 1 wt. %, the temperature of the bottom region being kept above the boiling point of the isobutyric acid and the temperature of the upper region being kept near the boiling point of the hydrogen fluoride, at the pressure present in each case.